# EUROPEAN PATENT APPLICATION

(11) **EP 4 442 698 A1**
(43) Date of publication of application: **09.10.2024**
(21) Application number: 22914735.0
(22) Date of filing: 27.12.2022
(51) Int. Cl.: C07K 14/015, C12N 7/00, C12N 15/35, C12N 15/864, A61K 48/00, A61K 38/00, A61K 35/76, A61P 27/02, A61P 19/02

(54) **MODIFIED AAV CAPSID PROTEIN AND USE THEREOF**

(30) Priority: 28.12.2021 CN 202111628667
(71) Applicant: Chengdu Origen Biotechnology Co., Ltd., Chengdu, Sichuan 610037 (CN)
(72) Inventor: KE, Xiao, Chengdu, Sichuan 610036 (CN); ZHENG, Qiang, Chengdu, Sichuan 610036 (CN); LUO, Shuang, Chengdu, Sichuan 610036 (CN); JIANG, Hao, Chengdu, Sichuan 610036 (CN)
(74) Representative: reuteler & cie SA
(86) International application number: PCT/CN2022/142185
(87) International publication number: WO 2023/125481

(57) **Abstract**

The present invention relates to a modified adeno-associated virus (AAV) capsid protein and use thereof; said capsid protein comprises substitution of approximately 5-14 amino acids with respect to a parent AAV capsid protein; furthermore, the AAV containing the modified capsid protein has increased infectiousness with respect to a target tissue or target cell (e.g. retinal cell) in comparison with an AAV comprising an unmodified parent AAV capsid protein.

## Description

### Field

The disclosure relates to the field of biotechnology, in particular to a modified AAV capsid protein and use thereof.

### Background

The vectors utilized in gene therapy can be classified into viral vectors and non-viral vectors. For viral vectors, those most commonly used are adenovirus vector, lentivirus vector, adeno-associated virus vector, herpes simplex virus vector and so on.

Adeno-associated viruses (AAVs) belong to *Parvoviridae* and *Dependoviruses.* The virion comprises a 25nm icosahedral capsid containing a 4.7kb single-stranded DNA genome with the two open reading frames below: Rep and Cap. The non-structural Rep gene encodes four kinds of regulatory proteins which are necessary for viral replication, while Cap encodes three types of structural proteins (VP1-3) forming 60-subunits capsids. The viral capsid mediates the abilities of AAV vectors to overcome many biological barriers to viral transduction, in which the biological barriers include cell surface receptor binding, endocytosis, intracellular transportation, and decapsulation in the nucleus.

AAVs have been widely used in fields of gene transduction, gene therapy, vaccination, oncolytic therapy and so on. It has been widely used in experimental and clinical researches. with lots of advantages, such as low pathogenicity, wide infectable tissue, wide range of host cells (can be infected and expressed in proliferating and non-proliferating cells), low immunogenicity, long *in vivo* expression time of foreign genes, and no integration, with host cell genome, The US FDA approved the first AAV-mediated gene therapy for treating a rare hereditary ophthalmopathy in 2017, in which the expression of a therapeutic drug delivered by a single subretinal administration of a rAAV vector was consistently observed for more than four years.

However, currently, there are still many challenges existing in the application of AAVs, such as how to improve the tissue and cell targeting ability and transduction efficiency of the carrier, and how to improve the packaging efficiency of a specific carrier, in order to adapt to industrial production.

### Summary

The disclosure relates to delivery of a viral vector, such as an AAV viral vector, containing a desired gene to a desired cell or tissue. Specifically, the disclosure comprises modified capsid protein with one or more modifications (such as substitution, insertion, or mutation) in the amino acid sequences compared to the parent AAV capsid protein. When present in an AAV virus vector, compared to an AAV virus vector containing an unmodified parent AAV capsid protein, the modified capsid protein has increased infectivity for target tissues or cells, such as the retina, muscles, or articular cavities.

In one aspect, the disclosure provides a modified adeno-associated virus (AAVs) capsid protein containing a polypeptide substitution of approximately 5-14 amino acids compared to the parent AAV capsid protein, and wherein AAVs including the modified AAV capsid protein have enhanced retinal cell infectivity compared to those including parent AAV capsid protein.

In some specific embodiments, the polypeptide comprises amino acid sequence selected from RGNRQ (SEQ ID NO: 1), QQNTARGNRQ (SEQ ID NO: 2), RGNRQAAQQNTA (SEQ ID NO: 3), RGNRQQNTA (SEQ ID NO: NO:4), RGNRQQQNTA (SEQ ID NO: 5), SGNTQ (SEQ ID NO: 6), RGNQQNTARQ (SEQ ID NO: 7), RGNQQPRPTSRQ (SEQ ID NO:8), RGNRQAAQQPTPTS (SEQ ID NO: 9) or RGNRQQQPTPTS (SEQ ID NO: 19); and the substitution is located at amino acid positions 588-592 of parent AAV8 or at the corresponding positions of another serotype capsid protein.

In some specific embodiments, the polypeptide of capsid protein comprises amino acid sequence selected from QQNTARGNRQ (SEQ ID NO: 2), RGNRQQNTA (SEQ ID NO: 4), SGNTQ (SEQ ID NO: 6), RGNQQNTARQ (SEQ ID NO: NO:7) or RGNRQQQPTPTS (SEQ ID NO: 19).

In some specific embodiments, the capsid protein further includes mutation at amino acids 262-272 of parent AAV8 capsid protein or corresponding positions of another serotype capsid protein, resulting in the mutated amino acid sequence of SQSGASNDNH (SEQ ID NO: 10). In a preferred embodiment, the capsid protein includes a polypeptide substitution at amino acid positions 588-592 of parent AAV8 or at the corresponding positions of another serotype capsid protein, wherein the polypeptide is RGNRQ (SEQ ID NO: 1); and a mutation at amino acids 262-272 of parent AAV8 capsid protein or corresponding positions of another serotype capsid protein, wherein the mutated amino acid sequence is SQSGASNDNH (SEQ ID NO: 10).

In some specific embodiments, the AAV in this disclosure may be derived from AAV of any serotype, for example, the AAV serotype is selected from AAV1, AAV2, AAV3, AAV4, AAV5, AAV6, AAV7, AAV8, AAV9, AAV10, AAV11, AAV12, AAV-DJ, AAV-DJ8, AAV-DJ9, AAVrh8, AAVrh8R, or AAVrh10. In a preferred embodiment, the AAV is this disclosure is selected from AAV8.

In some specific embodiments, the capsid protein further includes mutation at the amino acids site of D80 and/or V125 relative to the parent AAV8 capsid protein. In a preferred embodiment, the mutation is D80N and/or V125A. In another preferred embodiment, the mutation is D80Q and/or V125G. In a preferred embodiment, the capsid protein includes a polypeptide substitution at amino acid sites 588-592 of parent AAV8, in which the polypeptide is RGNQQNTARQ (SEQ ID NO: 7); and amino acid mutations at sites of D80 and V125 relative to parent AAV8 capsid proteins, wherein the mutations are D80N and V125A. In another preferred specific embodiment, the capsid protein includes a polypeptide substitution at amino acid sites 588-592 of parent AAV8, in which the polypeptide is RGNQQNTARQ (SEQ ID NO: 7); and amino acid mutations at sites of D80 and V125 relative to parent AAV8 capsid proteins, wherein the mutations are D80Q and V125G.

Another aspect of the disclosure provides another modified adeno-associated virus (AAV) capsid protein comprising a polypeptide insertion after amino acid site 589 of parent AAV8 or a corresponding position of capsid protein of another serotype, and when compared to AAV viruses containing the corresponding parent AAV capsid protein, those containing the modified capsid protein have enhanced retinal cell infectivity.

In some specific embodiments, the polypeptide comprises an amino acid sequence selected from RGDLTTPQQ (SEQ ID NO: 20), RGDLNTPQQ (SEQ ID NO: 21), or RGDVSSPQQ (SEQ ID NO: 22). The AAV can be derived from AAV of any serotype, for example, the AAV serotype is selected from AAV1, AAV2, AAV3, AAV4, AAV5, AAV6, AAV7, AAV8, AAV9, AAV10, AAV11, AAV12, AAV-DJ, AAV-DJ8, AAV-DJ9, AAVrh8, AAVrh8R, or AAVrh10. In a preferred embodiment, the AAV serotype is AAV8.

In the other aspect, the disclosure provides a recombinant adeno-associated virus (rAAV) comprising:
i. the modified capsid protein provided in this disclosure;
ii. heterologous nucleic acids comprising encoded gene products.

In some specific embodiments, the gene product is a VEGF antagonist or a TNF-α antagonist.

In some specific embodiments, the VEGF antagonist is selected from Aflibercept, Combercept, Ranibizumab, and Brolucizumab, preferred is selected from Aflibercept.

In some specific embodiments, the TNF-α antagonist is selected from Etanercept, Infliximab, Adalimumab, Pecelizumab, or Golimumab, preferred is selected from Etanercept.

In the other aspect, the disclosure provides a pharmaceutical composition, comprising:
a) the recombinant adeno-associated virus provided in this disclosure;
b) pharmaceutically acceptable excipients.

In the other aspect, the disclosure provides a recombinant adeno-associated virus according to the disclosure for use as a drug.

In some specific embodiments, the recombinant adeno-associated virus or pharmaceutical composition of the disclosure is administered by intravitreal, subretinal, suprachoroidal injection, by intravenous, subcutaneous, muscular, or joint cavity injection. In a preferred embodiment, the recombinant adeno-associated virus or pharmaceutical composition of the disclosure is administered by suprachoroidal space injection. In another preferred embodiment, the recombinant adeno-associated virus or pharmaceutical composition of the disclosure is administered by muscular or joint cavity injection.

In the other aspect, the disclosure provides recombinant adeno-associated viruses or pharmaceutical compositions according to the disclosure used for therapeutic purposes.

In some specific embodiments, the recombinant adeno-associated virus or pharmaceutical composition of the disclosure is administered via intravitreal, subretinal, or suprachoroidal injection. In a preferred embodiment, the recombinant adeno-associated virus or pharmaceutical composition of the disclosure is administered via suprachoroidal space injection.

In the other aspect, the disclosure provides the use of the recombinant adeno-associated virus in the preparation of drugs for the prevention or treatment of oculopathy.

In the other aspect, the disclosure provides a method for prevention or treatment of oculopathy, wherein the method comprises the administration of effective amounts of recombinant adeno-associated viruses or pharmaceutical compositions of the disclosure to individuals in need.

In some specific embodiments, the recombinant adeno-associated virus or drug composition described of the disclosure is administered via intravitreal, subretinal, or suprachoroidal injection. In a preferred embodiment, the recombinant adeno-associated virus or pharmaceutical composition of the disclosure is administered via suprachoroidal space injection.

In some specific embodiments, the oculopathy described in the disclosure is selected from retinal neovascularization, choroidal neovascularization, iridal neovascularization, corneal neovascularization oculopathy, non-infectious uveitis, or glaucoma. In some specific embodiments, the oculopathy described in the disclosure is selected from age-related macular degeneration, macular oedema, diabetic macular oedema, macular oedema secondary to retinal vein occlusion, retinal vein occlusion, central retinal vein occlusion, branch retinal vein occlusion, macular oedema caused by branch retinal vein occlusion, diabetic retinal oedema, diabetic retinopathy, proliferative diabetic retinopathy, diabetic retinal ischemia, polypoidal choroidal vasculopathy, choroidal neovascularization secondary to degenerative myopia, or retinopathy of prematurity.

In the other aspect, the disclosure provides the recombinant adeno-associated virus or pharmaceutical composition according to the disclosure for the treatment of oculopathy.

In some specific embodiments, the recombinant adeno-associated virus or pharmaceutical composition described in the disclosure is administered via intravitreal, subretinal, or suprachoroidal injection. In a preferred embodiment, the recombinant adeno-associated virus or pharmaceutical composition of the disclosure is administered via suprachoroidal space injection.

In some specific embodiments, the oculopathy described in the disclosure is selected from retinal neovascularization, choroidal neovascularization, iridal neovascularization, corneal neovascularization oculopathy, non-infectious uveitis, or glaucoma. In some specific embodiments, the oculopathy described in the disclosure is selected from age-related macular degeneration, macular oedema, diabetic macular oedema, macular oedema secondary to retinal vein occlusion, retinal vein occlusion, central retinal vein occlusion, branch retinal vein occlusion, macular oedema caused by branch retinal vein occlusion, diabetic retinal oedema, diabetic retinopathy, proliferative diabetic retinopathy, diabetic retinal ischemia, polypoidal choroidal vasculopathy, choroidal neovascularization secondary to degenerative myopia, or retinopathy of prematurity.

In the other aspect, the disclosure provides use of the recombinant adeno-associated virus in preparation of drugs for prevention or treatment of arthritic diseases or related conditions.

In the other aspect, the disclosure provides a method for prevention or treatment of arthritic diseases or related conditions, which comprises the administration of effective amount of recombinant adeno-associated virus or pharmaceutical composition according to the disclosure to individual in need.

In some specific embodiments, the recombinant adeno-associated virus or pharmaceutical composition described in the disclosure is administered via intravenous, subcutaneous, muscular, or joint cavity injection.

In some specific embodiments, the recombinant adeno-associated virus or pharmaceutical composition described in the disclosure is administered via muscular or joint cavity injection.

In some specific embodiments, the arthritic disease or related condition described in the disclosure is selected from rheumatoid arthritis, juvenile rheumatoid arthritis, osteoarthritis, gout, pseudogout, spondylitis, Crohn's disease, plaque parapsoriasis, psoriatic arthritis, ankylosing spondylitis, septic arthritis, arthritis, juvenile idiopathic arthritis, blunt trauma, joint replacement or Still's disease.

In the other aspect, the disclosure provides recombinant adeno-associated virus or pharmaceutical composition according to the disclosure for treatment of arthritic diseases or related conditions.

In some specific embodiments, the recombinant adeno-associated virus or pharmaceutical composition described in the disclosure is administered via intravenous, subcutaneous, muscular, or joint cavity injection.

In some specific embodiments, the recombinant adeno-associated virus or pharmaceutical composition described in the disclosure is administered via muscular or joint cavity injection.

In some specific embodiments, the arthritic disease or related condition described in the disclosure is selected from rheumatoid arthritis, juvenile rheumatoid arthritis, osteoarthritis, gout, pseudogout, spondylitis, Crohn's disease, plaque parapsoriasis, psoriatic arthritis, ankylosing spondylitis, septic arthritis, arthritis, juvenile idiopathic arthritis, blunt trauma, joint replacement or Still's disease.

### Brief Description of the Drawings

Figure 1A shows the amino acid sequence alignment of AAV capsid protein in part regions of GH ring;
Figure 1B shows the amino acid sequence alignment of AAV capsid protein in part regions of GH ring;
Figure 2 shows the schematic diagram of the construction of AAV8 capsid protein mutant plasmid;
Figure 3 shows the EGFP mRNA expression levels in mice with AAV8 mutant virus intravitreal injection;
Figure 4 shows the EGFP fluorescence signal of retinal patch from mice with AAV8 mutant virus via intravitreal injection;
Figure 5 shows the EGFP fluorescence signal of retinal patch from rats with AAV8 mutant virus via intravitreal injection;
Figure 6 shows the EGFP fluorescence signal and DAPI signal from rabbits with AAV8 mutant virus via suprachorioidal space injection;
Figure 7 shows the EGFP fluorescence signal of AAV8 mutant virus cultured in ARPE19 cells;
Figure 8A shows the EGFP positive cell count via flow cytometry of ARPE19 cells infected with AAV8 mutant virus.
Figure 8B shows the EGFP positive cell count via flow cytometry of ARPE19 cells infected with AAV8 mutant virus.
Figure 9 shows the relative genomic DNA content of mCherry form rabbits with AAV8 mutant virus via suprachorioidal space injection;
Figure 10 shows the luciferase fluorescence intensity of mice with AAV8 mutant virus via muscular injection;
Figure 11 shows the clinical score of mice with AAV8 mutant virus via joint cavity/muscular injection;
Figure 12 shows the thickness of sole of foot of mice with AAV8 mutant virus via joint cavity/ muscular injection.

### Detailed Description

The disclosure relates to capsid protein with increased infectivity for target tissues or target cells (e.g., retina, muscle, or joint cavity) compared to AAV viral vectors containing unmodified parent AAV capsid protein, rAAVs containing the capsid protein and expressing target gene (e.g., VEGF antagonists, TNF- α antagonists) and pharmaceutical composition thereof, and use thereof.

### Capsid Protein

The disclosure provides a modified AAV capsid protein, wherein the modified AAV capsid protein includes one or more amino acid modifications compared to the corresponding wild-type AAV or parent AAV. The modifications comprise substitutions, deletions, or insertions of one or more amino acids, compared to the parent capsid protein or AAV capsid protein. The modification has increased infectivity for target tissues or target cells, such as the retina, muscle, or joint cavity, compared to AAV viral vectors containing unmodified parent AAV capsid proteins.

In particular embodiments, the modified AAV capsid protein comprises hetero-peptide substitution in capsid protein serotypes such as VP1 belonging to: AAV Type 1, AAV Type 2, AAV Type 3, AAV Type 4, AAV Type 5, AAV Type 6, AAV Type 7, AAV Type 8, AAV Type 9, AAV Type 10, AAVrh10, avian AAV, bovine AAV, canine AAV, equine AAV, primate AAV, non-primate AAV, bovine AAV, AAV2.7m8, AAVShH10, AAV2.5T, AAV2.5T/7m8, AAV9/7m8, and AAV5/7m8. In some embodiments, the capsid variant may be a chimeric capsid variant. Sequence of chimeric capsid variant may contain parts of two or more AAV capsid serotypes or variant thereof. In some embodiments, chimeric capsid contains parts of 2, 3, 4, 5, 6, 7, 8, 9, 10 or more different capsid protein serotypes.

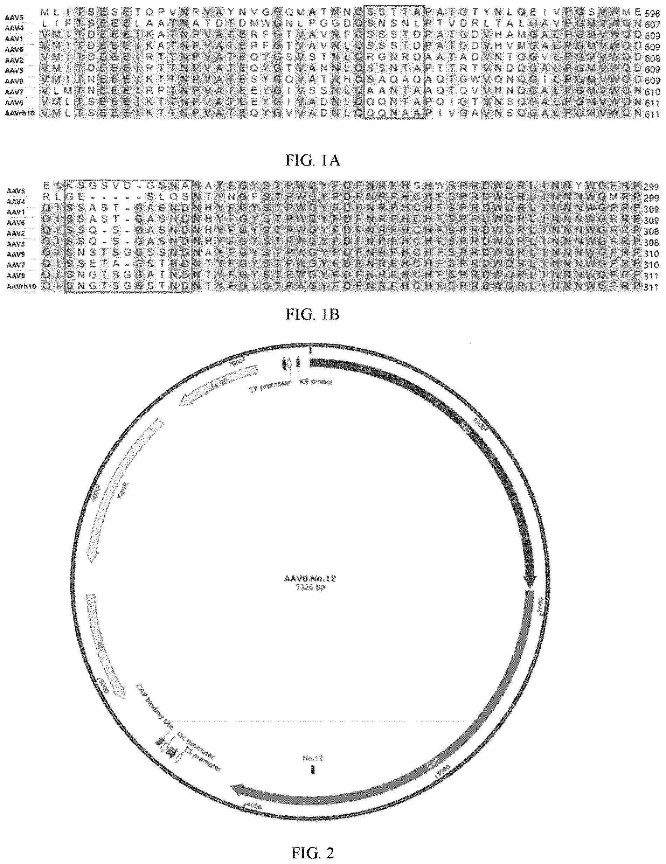

In particular embodiments, the modified AAV capsid protein comprises a heteropolypeptide substitution of approximately 5-14 amino acids. In some particular embodiments, the modified capsid protein of the disclosure comprises heteropolypeptide substitution of approximately 5-14 amino acids at amino acids 588-592 of parent AAV8 or at corresponding positions of another serotype capsid protein. The amino acid sequence of AAV1-AAV9 etc. described such as in patents US8962330B, US10041090B, etc. In a specific embodiment, the modified capsid protein of the disclosure comprises heteropolypeptide substitution of approximately 5-14 amino acids located at amino acid positions 588-592 of parent AAV8. The 588-592 amino acids of the parent/wild type AAV8 capsid protein are "QQNTA". Therefore, a specific embodiment of the disclosure is the modified capsid protein with substitution of the parent/wild-type AAV8 capsid protein at sites 588-592 "QQNTA" for a heteropolypeptide with 5-14 amino acids. The substitution sites corresponding to amino acids 588-592 of AAV8 are at the corresponding positions of other capsid proteins, e.g. as shown in Figure 1A. For example, at 586-590 amino acids of AAV1, at 585-589 amino acids of AAV2, at 586-590 amino acids of AAV3, at 584-588 amino acids of AAV4, at 575-579 amino acids of AAV5, at 586-590 amino acids of AAV6, at 587-591 amino acids of AAV7, at 586-590 amino acids of AAV9, at 588-592 amino acids of AAV10,.

The length of the above substituting heteropolypeptide is 5 amino acids, 6 amino acids, 7 amino acids, 8 amino acids, 9 amino acids, 10 amino acids, or 11 amino acids. In some specific embodiments, the heteropolypeptide of the disclosure comprises amino acid sequence selected from RGNRQ (SEQ ID NO: 1), QQNTARGNRQ (SEQ ID NO: 2), RGNRQAAQQNTA (SEQ ID NO: 3), RGNRQQNTA (SEQ ID NO: NO:4), RGNRQQQNTA (SEQ ID NO: 5), SGNTQ (SEQ ID NO: 6), RGNQQNTARQ (SEQ ID NO: 7), RGNQQPRPTSRQ (SEQ ID NO:8), RGNRQAAQQPTPTS (SEQ ID NO: 9), or RGNRQQQPTPTS (SEQ ID NO: 19). In some preferred embodiments, the polypeptide of the disclosure comprises amino acid sequence selected from QQNTARGNRQ (SEQ ID NO: 2), RGNRQQNTA (SEQ ID NO: 4), SGNTQ (SEQ ID NO: 6), or RGNQQNTARQ (SEQ ID NO: 7), or RGNRQQQPTPTS (SEQ ID NO: 19).

In particular embodiments, the modified AAV capsid protein further includes mutations at amino acids 262-272 compared to the parent AAV8 capsid protein or at the corresponding location of another serotype capsid protein. Amino acids 262-272 of the parent/wild-type AAV8 capsid protein are "SNGTSGGATND". Therefore, a specific embodiment of the disclosure is that the modified capsid protein is mutated at "SNGTSGGATND" of amino acids sites 262-272 of the parent/wild type AAV8 capsid protein. The mutation sites corresponding to amino acids 262-272 of AAV8 are at the corresponding positions of other capsid proteins, such as amino acids 261-271 of AAV1, amino acids 261-271 of AAV2, amino acids 261-271 of AAV3, amino acids 255-265 of AAV4, amino acids 251-261 of AAV5, amino acids 261-271 of AAV6, amino acids 262-272 of AAV7, amino acids 261-271 of AAV9, amino acids 262-272 of AAV10. The mutation sites corresponding to amino acids 262-272 of AAV8 are at the corresponding locations of other capsid proteins, e.g. as shown in Figure 1B. In some specific embodiments, the mutated amino acid sequence of the disclosure is SQSGASNDNH (SEQ ID NO: 10).

In some specific embodiments, the modified capsid protein of the disclosure comprises a polypeptide substitution at amino acid sites 588-592 of parent AAV8 or at the corresponding position of another serotype capsid protein, in which the polypeptide is RGNRQ (SEQ ID NO: 1); and a mutation at amino acid sites 262-272 of parent AAV8 capsid protein or corresponding positions of another serotype capsid protein, in which the mutated amino acid sequence is SQSGASNDNH (SEQ ID NO: 10). In some preferred embodiments, the parent capsid protein serotype of the disclosure is AAV8.

In other embodiments, the modified AAV capsid protein in the disclosure further includes amino acid mutation at sites of D80 and/or V125 relative to the parent AAV8 capsid protein. In some specific embodiments, the mutation is D80N and/or V125A. In some specific embodiments, the mutation is D80Q and/or V125G. In some preferred embodiments, the capsid protein includes a polypeptide substitution at amino acid sites 588-592 of parent AAV8, in which the polypeptide is RGNQQNTARQ (SEQ ID NO: 7); and amino acid mutations at sites of D80 and V125 relative to parent AAV8 capsid proteins, in which the mutations are D80N and V125A. In other preferred embodiments, the capsid protein includes a polypeptide substitution at amino acid sites 588-592 of parent AAV8, in which the polypeptide is RGNQQNTARQ (SEQ ID NO: 7); and amino acid mutations at sites of D80 and V125 relative to parent AAV8 capsid proteins, in which the mutations are D80Q and V125G.

In other embodiments, the modified AAV capsid protein in the disclosure comprises a polypeptide insertion after amino acid site 589 of parent AAV8 or the corresponding position of another serotype capsid protein. Insertion site is located after amino acid site 589 of AAV8 or corresponding positions of other capsid proteins, such as after 589 amino acid of AAV1, 586 amino acid of AAV2, 587 amino acid of AAV3, 585 amino acid of AAV4, 576 amino acid of AAV5, 587 amino acid of AAV6, 588 amino acids of AAV7, 587 amino acids of AAV9, 589 amino acids of AAV10. In some specific embodiments, the inserted polypeptide contains an amino acid sequence of RGDLTTPQQ (SEQ ID NO: 20), RGDLNTPQQ (SEQ ID NO: 21), or RGDVSSPQQ (SEQ ID NO: 22). In a preferred embodiment, the modified AAV capsid protein in the disclosure comprises a polypeptide insertion after amino acid position 589 relative to parent AAV8, in which the polypeptide comprises an amino acid sequence selected from RGDLTTPQQ (SEQ ID NO: 20), RGDLNTPQQ (SEQ ID NO:21) or RGDVSSPQQ (SEQ ID NO: 22).

In some embodiments, the modified capsid protein in the disclosure further comprises amino acid sequence with at least 85%, 90%, 95%, 98%, 99%, or 100% homology to the capsid protein mentioned above that has been substituted, mutated, and/or inserted.

In some embodiments, AAV virus containing modified capsid protein has increased infectivity to retinal cells by at least 1, 2, 5, 10, 20, 50, 100, or even more fold compared with AAV virus containing parental/wild-type capsid proteins. The retinal cells include RPE-19 cells (human retinal pigment epithelium cell line), retinal ganglion cells, amacrine cells, horizontal cells, bipolar cells, photoreceptor cells, cone cells, rod cells, Müller glial cell and retinal pigment epithelium cells, etc.

In some specific embodiments, AAV virus containing modified capsid protein has increased infectivity to RPE cells by at least 1, 2, 5, 10, 20, 50, 100 or even more fold compared to AAV viruses containing parental/wild-type capsid proteins.

In some specific embodiments, compared to AAV viruses containing parental/wild-type capsid proteins, AAV virus containing modified capsid protein has increased infectivity to retinal cells by at least 1, 2, 5, 10, 20, 50, 100 or even more times after suprachorioidal space injection.

In some specific embodiments, compared to AAV viruses containing parental/wild-type capsid proteins, AAV virus containing modified capsid protein has increased infectivity to retinal cells by at least 1, 2, 5, 10, 20, 50, 100 or even more times after intravitreal injection.

### Recombinant Adeno-Associated Virus (rAAVs)

The gene delivery vector in the disclosure is recombinant adeno-associated virus (rAAVs). In some embodiments, the rAAV in the disclosure is a single-chain AAV (ssAAV). ssAAV refers to a rAAV that has the coding sequence and complementary sequence of the target gene on a separate strand and that is packaged in a separate viral capsid.

In some embodiments, the recombinant adeno-associated virus (rAAV) in the disclosure includes: i) a modified capsid protein according to the disclosure; ii) heterologous nucleic acids comprising encoded gene products.

In some embodiments, the rAAV in the disclosure includes the modified capsid protein of the disclosure above, such that the recombinant AAV (rAAV) in the disclosure has enhanced cell or tissue (e.g., eye tissue or cells, muscle tissue or joint cavity, etc.) specific targeting ability or infectivity relative to rAAVs containing parent or wild-type capsid proteins.

In some embodiments, the rAAV in the disclosure comprises a polynucleotide expression cassette comprising a heterologous nucleic acid encoding a gene product and a regulatory element. The term "regulatory element" refers to a nucleic acid sequence that regulates the expression of a gene product to which it is operationally linked. Thus, expression regulatory element may include promoters, enhancers, internal ribosome entry sites (IRES), transcriptional terminators, start codons before protein-coding genes, intron splicing signals, and stop codons. The term may also include nucleic acid sequence design that removes undesired possible start codons, both inside and outside the frame, from the sequence. It may also include nucleic acid sequence design to remove undesired possible splicing sites. It may also include a sequence directing the addition of polyadenylation or polyA, etc.

In the polynucleotide expression cassette of the disclosure, the heterologous nucleic acid includes a nucleotide sequence encoding a gene product of a target gene, such as a therapeutic gene product. In some embodiments, the gene product is an interfering RNA. In some embodiments, the gene product is an aptamer. In some embodiments, the gene product is a polypeptide. In some embodiments, the gene product is a site-specific nuclease that provides site-specific knockdowns of gene function. In some preferred embodiments, the gene product is a polypeptide, a protein, a fusion protein, an antibody, etc.

In some embodiments, the gene product in the disclosure is selected from VEGF antagonist, TNF-α antagonist, PD-1/PD-L1 inhibitor, Ang-2 inhibitor, plasma kallikrein inhibitor, endostatin, tumorstatin, angiostatin, pigment epithelium-derived factor (PEDF), soluble Tie-2 receptor, Ang-2 antagonist, CD tissue inhibitor of metalloproteinase 3 (TIMP-3), photoresponsive opsin (e.g., rhodopsin), anti-apoptotic polypeptide (e.g., Bcl-2, Bcl-Xl), glia-derived neurotrophic factor (GDNF), fibroblast growth factor 2, neurturin, NTN, ciliary neurotrophic factor (CNTF), nerve growth factor (NGF), neurotrophin-4 (NT4), brain-derived neurotrophic factor (BDNF) and functional variant and fragment thereof, variant with at least 80%, at least 85%, at least 90%, or at least 95% sequence identity of any protein or polypeptide in these proteins or polypeptides, and fragment comprising at least 20%, at least 30%, at least 50%, at least 60%, at least 70%, at least 80%, or at least 90% of these proteins, polypeptides, or variants thereof.

In a embodiment, the gene product in the disclosure is selected from a VEGF antagonist. In a specific embodiment, the VEGF antagonist is selected from any one of the extracellular domains 1-7 of VEGF receptor 1 (VEGFR-1, or Flt) or receptor 2 (VEGFR-2, or Flk), for example, VEGFR-1 extracellular domain 2 (Fit d2) and/or VEGFR-2 extracellular domain 3 or 4.

In a specific embodiment, the VEGF antagonist comprises at least one selected from the following groups:
a) Fusion protein comprising extracellular domain 2 of VEGFR-1 and extracellular domain 3 of VEGFR-2; as an example, the fusion protein comprises an amino acid sequence as setting forth in SEQ ID NO: 11 (Fltd2 Flkd3);
b) Fusion protein comprising extracellular domain 2 of VEGFR-1, extracellular domain 3 and domain 4 of VEGFR-2; as an example, the fusion protein comprises an amino acid sequence as setting forth in SEQ ID NO: 12 (Fltd2 Flkd3, 4);
c) with an antibody heavy chain variable region sequence as setting forth in SEQ ID NO: 13 and an antibody light chain variable region sequence as setting forth in SEQ ID NO: 14;
d) with an antibody heavy chain variable region sequence as setting forth in SEQ ID NO: 15 and an antibody light chain variable region sequence as setting forth in SEQ ID NO: 16.

Wherein, SEQ ID NO: 12 is the sequence as setting forth below: 
GRPFVEMYSEIPEIIHMTEGRELVIPCRVTSPNITVTLKKFPLDTLIPDGKRIIWDSRKGF
IISNATYKEIGLLTCEATVNGHLYKTNYLTHRQTNTIIDVVLSPSHGIELSVGEKLVLN
CTARTELNVGIDFNWEYPSSKHQHKKLVNRDLKTQSGSEMKKFLSTLTIDGVTRSDQ
GLYTCAASSGLMTKKNSTFVRVHEKPFVAFGSGMESLVEATVGERVRIPAKYLGYPP
PEIKWYKNGIPLESNHTIKAGHVLTIMEVSERDTGNYTVILTNPISKEKQSHVVSLVVY
VPPGPGDKTHTCPLCPAPELLGGPSVFLFPPKPKDTLMISRTPEVTCVVVDVSHEDPEV
 
KFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQ
PENNYKATPPVLDSDGSFFLYSKLTVDKSRWQQGNVFSCSVMHEALHNHYTQKSLS
LSPGK
SEQ ID NO: 13 is the sequence as setting forth below:
SEQ ID NO: 14 is the sequence as setting forth below: 
DIQLTQSPSSLSASVGDRVTITCSASQDISNYLNWYQQKPGKAPKVLIYFTSSLHSGVP
SRFSGSGSGTDFTLTISSLQPEDFATYYCQQYSTVPWTFGQGTKVEIKRTVAAPSVFIF
PPSDEQ
SEQ ID NO: 15 is the sequence as setting forth below: 
EVQLVESGGGLVQPGGSLRLSCTASGFSLTDYYYMTWVRQAPGKGLEWVGFIDPDD
DPYYATWAKGRFTISRDNSKNTLYLQMNSLRAEDTAVYYCAGGDHNSGWGLDIWG
QGTLVTVSS
SEQ ID NO: 16 is the sequence as setting forth below:

MEIVMTQSPSTLSASVGDRVIITCQASEIIHSWLAWYQQKPGKAPKLLIYLASTLASGV
PSRFSGSGSGAEFTLTISSLQPDDFATYYCQNVYLASTNGANFGQGTKLTVLG

In a specific embodiment, the VEGF antagonist has an amino acid sequence as setting forth in SEQ ID NO: 11:

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKL
VLNCTARTELNVGIDFNWEYPSSKHQHKKLVNRDLKTQSGSEMKKFLSTLTIDGVTR
SDQGLYTCAASSGLMTKKNSTFVRVHEKDKTHTCPPCPAPELLGGPSVFLFPPKPKDT
LMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 11)

In a specific embodiment, the nucleotide encoding the VEGF antagonist has a sequence as setting forth in SEQ ID NO: 23:

AGCGACACCGGCCGGCCCTTCGTGGAGATGTACAGCGAAATCCCTGAGATCATTC
ACATGACAGAAGGCCGCGAACTGGTGATTCCCTGCAGGGTTACCAGCCCAAACA
TCACCGTGACCCTGAAGAAGTTCCCCCTGGACACCCTGATCCCTGACGGCAAACG
GATCATCTGGGACTCTAGGAAAGGCTTTATCATCTCCAATGCCACATACAAAGAG
ATTGGCCTGCTGACCTGCGAGGCTACGGTCAACGGCCACCTGTACAAAACAAATT
ACCTGACCCACAGACAGACCAACACAATCATCGACGTGGTGCTGAGCCCATCCCA
TGGCATTGAGCTGTCCGTGGGAGAAAAGCTGGTGCTGAATTGTACGGCTAGAACA
GAACTTAACGTGGGCATCGATTTCAACTGGGAGTACCCTAGCTCCAAGCACCAGC
ATAAAAAGCTGGTGAACCGGGACCTGAAGACCCAGTCCGGCAGTGAGATGAAAA
AGTTCTTGAGCACCCTGACCATCGATGGAGTGACCCGAAGCGACCAGGGCCTGTA
CACCTGTGCCGCCTCCTCTGGGCTGATGACAAAAAAGAACAGCACCTTTGTCAGA
GTGCACGAGAAGGATAAAACCCACACCTGCCCTCCTTGTCCCGCTCCTGAGCTGC
TGGGGGGACCATCTGTGTTCCTGTTCCCCCCAAGCCCAAGGATACCCTGATGAT
CAGCCGAACCCCAGAAGTGACCTGCGTGGTGGTGGACGTGTCCCACGAGGACCC

AGAGGTGAAATTCAACTGGTATGTGGACGGCGTGGAGGTGCACAATGCCAAGAC
CAAACCAAGAGAGGAGCAGTATAACTCCACCTACAGGGTGGTGAGCGTGCTGAC
TGTGCTGCACCAGGATTGGCTGAACGGCAAGGAGTACAAGTGCAAGGTTAGCAA
TAAAGCTCTGCCCGCCCCCATCGAAAAGACCATTTCCAAAGCCAAAGGGCAGCC
AAGGGAGCCCCAGGTGTACACCCTGCCCCCATCTCGGGACGAGCTGACCAAGAA
CCAGGTGAGTCTCACATGTCTCGTGAAGGGCTTTTACCCTAGCGACATCGCCGTC
GAGTGGGAAAGCAACGGACAGCCTGAGAATAACTATAAGACCACACCCCCCGTG
CTGGATTCCGACGGCAGTTTTTTCCTGTACTCTAAGCTGACAGTGGACAAATCCC
GCTGGCAGCAGGGCAACGTGTTCAGCTGCTCCGTGATGCACGAGGCCCTGCACAA
TCACTATACACAGAAGAGCCTGAGCCTGAGCCCCGGGAAGTGA (SEQ ID NO: 23)

In some embodiments, the protein or polypeptide is selected from a TNF-α antagonist. In some specific embodiments, the VEGF antagonist comprises those selected from Etanercept, Infliximab, Adalimumab, Pecelizumab, or Golimumab.

In a specific embodiment, the TNF-α antagonist has a heavy chain variable region sequence as setting forth in SEQ ID NO: 17 and a light chain variable region sequence as setting forth in SEQ ID NO: 18.
SEQ ID NO: 17 is the sequence as setting forth below:
SEQ ID NO: 18 is the sequence as setting forth below:

In a specific embodiment, the TNF-α antagonist has the amino acid sequence as setting forth in SEQ ID NO: 24:

LPAQVAFTPYAPEPGSTCRLREYYDQTAQMCCSKCSPGQHAKVFCTKTSDTVCDSCE
DSTYTQLWNWVPECLSCGSRCSSDQVETQACTREQNRICTCRPGWYCALSKQEGCR
LCAPLRKCRPGFGVARPGTETSDVVCKPCAPGTFSDTTSSTDICRPHQICNVVAIPGDA
SMDAVCTSTSPTRSMAPGAVHLPQPVSTRSQHTQPTPEPSTAPSTSFLLPMGPSPPAEG
STGDEPKSCDKTHTCPPCPAPELLGGPSVFLFPPKPKDTLMISRTPEVTCVVVDVSHED
PEVKFNWYVDGVEVHNAKTKPREEQYDSTYRVVSVLTVLHQDWLNGKEYKCKVSN
KALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQK
SLSLSPG (SEQ ID NO:24)

In a specific embodiment, the nucleotide encoding the TNF-α antagonist has a sequence as setting forth in SEQ ID NO: 25:

CTGCCCGCCCAAGTGGCCTTCACCCCCTATGCCCCCGAGCCCGGCTCCACCTGTA
GACTGAGAGAGTACTACGATCAGACCGCTCAGATGTGCTGCAGCAAGTGCAGCC
CCGGGCAGCACGCCAAGGTGTTCTGCACCAAGACAAGCGACACCGTGTGCGACA
GCTGCGAGGACAGCACATACACACAACTGTGGAACTGGGTGCCCGAGTGCCTGA
GCTGCGGCAGCAGATGCAGCAGCGACCAAGTGGAGACCCAAGCCTGCACAAGAG
AGCAGAACAGAATCTGCACCTGCAGACCCGGCTGGTACTGCGCCCTGAGCAAGC
AAGAGGGCTGCAGACTGTGCGCCCCCCTGAGAAAGTGTAGACCCGGCTTTGGCGT
GGCTAGACCCGGCACAGAAACAAGCGACGTCGTGTGCAAACCTTGCGCTCCCGG
CACCTTCAGCGACACCACAAGCAGCACCGACATCTGCAGACCCCATCAGATCTGC

AACGTGGTGGCCATCCCCGGCGACGCTAGCATGGACGCCGTCTGCACCTCCACAA
GCCCCACCCGGAGCATGGCTCCTGGCGCCGTGCATCTGCCTCAGCCCGTGAGCAC
CCGGTCCCAACACACACAGCCTACCCCCGAGCCTAGCACCGCTCCCTCCACATCC
TTCCTGCTCCCTATGGGGCCTAGCCCCCCTGCCGAAGGCAGCACCGGCGACGAGC
CCAAGAGCTGCGATAAAACCCACACATGCCCCCCCTGCCCCGCCCCTGAACTCCT
GGGCGGGCCTAGCGTGTTCCTGTTCCCCCCCAAGCCCAAGGACACCCTGATGATC
AGCAGAACCCCCGAGGTGACCTGCGTGGTCGTGGACGTGAGCCACGAGGACCCC
GAGGTGAAGTTCAACTGGTACGTGGACGGCGTGGAGGTGCACAACGCCAAGACC
AAGCCTAGAGAGGAGCAGTACGACAGCACCTACAGAGTCGTGAGCGTGCTGACC
GTGCTGCACCAAGACTGGCTGAACGGCAAGGAGTACAAGTGCAAGGTGAGCAAC
AAGGCCCTGCCCGCTCCTATCGAGAAGACCATCAGCAAGGCCAAGGGGCAGCCT
AGAGAGCCCCAAGTGTACACCCTGCCCCCTAGCAGAGAGGAGATGACCAAGAAC
CAAGTGAGCCTGACCTGCCTGGTGAAGGGCTTCTACCCTAGCGACATCGCCGTGG
AGTGGGAGAGCAACGGGCAGCCCGAGAACAACTACAAGACCACCCCCCCCGTGC
TGGACAGCGACGGCAGCTTCTTCCTGTACAGCAAGCTGACCGTGGACAAGAGCA
GATGGCAGCAAGGCAACGTGTTCAGCTGCAGCGTGATGCACGAGGCCCTGCACA
ACCACTACACACAGAAGAGCCTGAGCCTGAGCCCCGGCTGA (SEQ ID NO: 25)

In some emboditions, the VEGF antagonist or TNF-α antagonist has an amino acid sequence that is at least 90%, at least 95%, at least 96%, at least 97%, at least 98%, at least 99% or 100% identical to the amino acid sequence above.

In some embodiments, the VEGF antagonist or TNF-α antagonist has a nucleotide sequence that is at least 90%, at least 95%, at least 96%, at least 97%, at least 98%, at least 99% or 100% identical to the nucleotide sequence above.

In some embodiments, the protein or polypeptide is selected from those having one or more distinct antigen-binding sites. For example, in some embodiments, the protein or polypeptide binds to both of VEGF and Ang-2. In a specific embodiment, the protein or peptide is a bis-specific antibody that binds to both of VEGF and Ang-2, such as Faricimab double-antibody.

In some embodiments of the disclosure, codon optimization can be used to design coding sequences for better expression of target genes. The invention can optimize nucleotide codons encoding polypeptide, fusion protein, antibody or functional fragment thereof to improve the protein expression level of the target gene in the target cell, tissue or species. The coding sequence is a part of the mRNA sequence that encodes amino acids for transcription and translation. During transcription and translation, every three of the 61 nucleotide codons are transcribed and translated into any one of the 20 amino acids. However, different cell types and different animal species encode the same amino acid for tRNA at different frequencies. When gene sequence contains infrequently expressed codons, ribosomal transcription mechanisms may be slowed down, resulting in prevention of effective transcription. Therefore, those skilled in this field can improve the expression level of target genes through "codon optimization". In short, the nucleotide sequence encoding the product is modified with synonymous codon sequences, such as replacing the codon with a lower frequency expression by a codon that can be expressed at a higher frequency in the target cell, so as to improve the expression level of the target substance in a specific cell, tissue or substance, such as increasing by 10%, 20%, 30%, 40%, 50%, 60%, 70%.80%, 90%, 100% or more. Codon-optimized coding sequences can be designed in a variety of ways. This optimization can be done using methods available online, publicly available methods, or companies that provide codon optimization services. For example, a codon optimization method is described in International Patent Publication WO2015/012924, which is incorporated herein by reference. The entire length of the product's open reading frame (ORF) is modified appropriately. However, in some embodiments, only a fragment of the ORF can be changed. By using one of these methods, the codon that encodes the polypeptide is generated to optimize the encoding nucleotide.

In some embodiments, the polynucleotide expression cassette of the present invention comprises one or more expression regulatory elements. For example, the regulatory element comprises a constitutive promoter region where the nucleotide sequence encoding the gene product is operably linked to a constitutive promoter, which is a promoter that is widely present and active in a cell, tissue, or species, and which promotes the expression of a target gene in a specific cell or tissue in vivo or in vitro. In some embodiments, Example of promoter comprises chicken β-actin promoter (CBA), cytomegalovirus promoter (CMV), CMV early enhancer/chicken β-actin promoter/rabbit β-globin intron (CAG), elongation factor 1α promoter (EF1α), human phosphoglycerate kinase promoter (PGK), MNT promoter, UB6 promoter, CAG promoter, RPE65 promoter, opsin promoter, mitochondrial heavy chain promoter, ubiquitin promoter, etc.

In some embodiments, the regulatory element comprises an inducible promoter region where the nucleotide sequence encoding the gene product is operably linked to the inducible promoter. In some embodiments, the inducible promoter is a photoreceptor specific promoter. Suitable photoreceptor specific promoter includes, for example, rhodopsin promoter, rhodopsin kinase promoter, β phosphodiesterase gene promoter, retinitis pigmentosa gene promoter, photoreceptor interretinoic acid-binding protein (IRBP) gene enhancer, IRBP gene promoter, opsin gene promoter, retinal splitting protein gene promoter, CRX homeodomain protein gene promoter, guanine nucleotide-binding protein alpha transduction active polypeptide 1 (GNAT1) gene promoter, neuroretinal specific leucine zipper protein (NRL) gene promoter, human cone arrestin (hCAR) promoter, and PR2.1, PR1.7, PR1.5, and PR1.1 promotor, RPE specific promoter (e.g. RPE65 gene promoter), cellular retinaldehyde-binding protein (CRALBP) gene promoter, pigment epithelium-derived factor (PEDF aka serpin)F1) gene promoter, vitelline macular dystrophy (VMD2) promoter, Müller glia cell-specific promoter (such as glial fibrillary acidic protein (GFAP) promoter), bipolar specific promoter (such as GRM6 promoter), etc.

The expression regulatory sequence of the polynucleotide expression cassette in the disclosure may also comprise a polyadenylation signal. The polyadenylation signal is also known as a polyadenylation site, a polyadenylation tail, a Poly(A) site, a Poly(A) signal, or a Poly(A) tail. The polyadenylation region refers to the covalent linkage of polyadenylate with messenger RNA (mRNA) molecules. In the process of protein biosynthesis, this is part of the way to produce mature mRNA ready for translation. In eukaryotes, polyadenylation is a mechanism by which mRNA molecules are interrupted at their 3'-terminal. Polyadenylation signal protects mRNAs from exonuclease attacks and are important for transcription termination, mRNA export from the nucleus, and translation. The polyadenylation signal comprises multiple consecutive adenosine monophosphates, which usually comprise AAUAAA repeats. Some examples of polyadenylation signal include monkey vacuolationd virus 40 (SV40), human growth hormone (HGH), bovine growth hormone (BGH), or beta-globin.

The polynucleotide expression cassette in the disclosure may also comprise an inverted terminal repeat sequence (ITR). Functional adenovirus reverse terminal repeat (ITR) refers to ITR sequences such as those used to integrate, replicate, and package AAV virions. Typically, the length of an ITR sequence is about 145bp. Preferably, essentially the entire sequence encoding the ITR is used in the molecule, together with modifications to these ITR sequences by those skilled in the art according to the usual technical means of the art. An example of the use of ITR molecules in this application is a "cis" plasmid containing the target gene, where the target gene sequence and associated regulatory elements are sidelined to the 5' and 3'AAV ITR sequences. AAV ITR sequences can be obtained from any known AAV, including currently identified mammalian AAV types. In some embodiments, the heterologous nucleotide encoding the target gene is on the side of the AAV ITR (e.g., in the direction 5 '-ITR-target gene-ITR-3'). In some embodiments, AAV ITR is selected from the group consisted of AAV1 ITR, AAV2 ITR, AAV3 ITR, AAV4 ITR, AAV5 ITR, AAV6 ITR, AAV7 ITR, AAV8 ITR, AAV9 ITR, AAV10 ITR, AAV 11 ITR and AAV 12 ITR.

The polynucleotide expression cassette in the disclosure further comprises an RNA output signal located downstream of the coding sequence and upstream of the polyadenylation site. RNA output signal is cis-acting post-transcriptional regulatory element that enhances RNA output from the nucleus. Examples of RNA output sequence include, but are not limited to, sequence from hepatitis B virus post-transcriptional regulatory elements (HPRE) and woodchuck hepatitis virus post-transcriptional regulatory elements (WPRE).

It is understandable for those skilled in the art that the polynucleotide expression cassette of the present invention may optionally comprise other elements, including, but not limited to, restriction sites that facilitate cloning, for operably connecting elements, or regulatory elements that regulate gene expression. Such as the bacterial sequence of plasmid vector, attp site, attB site, promoter junction, polyadenylation sequence junction of phage integrase vector, etc.

In some embodiments, the polynucleotide expression cassette in the disclosure comprises a polynucleotide expression cassette produced by a person skilled in the art by combining two or more elements of the above mentioned element and the target gene, where the target gene coding sequence and the associated regulatory element in the polynucleotide expression cassette are flanking 5' and 3'AAV ITR sequences.

For example, the polynucleotide expression cassette of the present invention may comprise, in order of 5' to 3', an N-terminal AAV ITR, an enhancer/promoter/intron, a Kozak sequence, a gene product coding sequence, a WPRE or HPRE RNA output signal sequence, a polyadenylation signal sequence, and a C-terminal AAV ITR.

In a specific embodiment, the recombinant adeno-associated virus in the disclosure comprises a polynucleotide expression cassette encoding the gene product, in which the sequence of the polynucleotide expression cassette from 5' to 3' terminals comprising:
(a) 5'AAV ITR;
(b) CMV enhancer;
(c) CBA promoter;
(d) chicken beta-actin intron;
(e) gene product coding sequence;
(f) rabbit β-globin polyadenylation signal sequence;
(g) 3'AAV ITR.

The gene product coding sequence is a nucleic acid sequence encoding the gene product above, such as the aforementioned VEGF antagonist or TNF-α antagonist.

Recombinant viral vectors containing the modified capsid proteins in the disclosure can be produced by using standard methods. For example, the method comprises culturing a host cell containing an artificial genome, which comprises: a cis-polynucleotide expression cassette side-attached by an AAV ITR, wherein the cis-polynucleotide expression cassette comprises a coding sequence of the gene product that is operatively attached to an expression control element that will control the expression of the gene product in human cells; A trans-expression cassette lacking AAV ITR, wherein the trans-expression cassette encodes the AAV rep and capsid protein, the AAV rep and capsid protein are operably attached to drive the expression of the AAV rep and capsid protein in the host cell in the culture and trans-supply the expression control elements of the rep and cap proteins; adenovirus auxiliary functions sufficient to permit replication and packaging of the artificial genome via the AAV capsid protein; and recycling the recombinant AAV packaging the artificial genome from the cell culture.

"Host cell" refers to any cell that contains or is capable of containing a target substance. Typically, the host cell is mammalian cells. In some embodiments, the host cell is photoreceptor cell, retinal pigment epithelial cell, keratinocyte, corneal cell, and/or tumor cell.

### Pharmaceutical composition

Another aspect of the disclosure is to provide a pharmaceutical composition comprising rAAV, wherein the composition comprises the recombinant adeno-associated virus described above and a pharmaceutically acceptable diluent, carrier or excipient.

A person skilled in the art can easily select a suitable diluent, carrier or excipient. As used herein, a carrier or excipient includes any solvent, dispersion medium, carrier, coating, diluent, antibacterial and antifungal agent, isotonic agent, absorption-delaying agent, buffer, carrier solution, suspension, colloid, preservative or chemical stabilizer.

Examples of carriers or excipients include sterile saline, lactose, sucrose, calcium phosphate, gelatin, dextran, agar, pectin, peanut oil, sesame oil, and water. For example, a suitable excipient or carrier includes saline that can be blended with a variety of buffer solutions, such as phosphate buffers. The action of microorganisms can be prevented by various antibacterial and antifungal agents, such as parabens, chlorobutanol, phenol, sorbate, thiomersal, etc. In many cases, it is best to include isotonic agents, such as saccharide or sodium chloride. The absorption of injectable compositions can be prolonged by using reagents that delay absorption, such as aluminum monostearate and gelatin, in the composition.

In order to give an injectable aqueous solution, a buffer solution can be properly formulated as needed, and the liquid diluent can first be isotonic with sufficient saline or glucose. These specific aqueous solutions are particularly suitable for intravenous, intramuscular, subcutaneous and peritoneal administration.

A sterile injection solution is prepared by mixing the required amount of active rAAV with various other ingredients enumerated in the present invention in an appropriate solvent and then filtering and sterilizing. Typically, dispersions are prepared by incorporating various sterilized active ingredients into a sterile carrier containing the dispersing medium and other required ingredients.

The rAAV composition in the disclosure can also be formulated in neutral or salt form. Pharmaceutically acceptable salts, including acid addition salts and formed from inorganic acids (e.g. hydrochloric or phosphoric acid) or organic acids (e.g. acetic acid, oxalic acid, tartaric acid, mandelic acid). Salts formed from free carboxyl groups can also be derived from inorganic bases, such as sodium, potassium, ammonium, calcium, or ferric hydroxide, or organic bases, such as isopropylamine, trimethylamine, histidine, procaine, etc. When the formulation is finished, the solution will be administered in a manner compatible with the dosage form and in a therapeutic effective amount. The formulation is readily administered in various dosage forms, such as injectable solutions, drug release capsules, etc.

The composition in the disclosure can be delivered to a suitable host cell by delivery carriers such as liposomes, nanocapsules, particles, microspheres, lipid particles, vesicles, etc.

It is advantageous to formulate injectable, oral or parenteral compositions in unit dosage forms for ease of administration. Unit dosage form, as used herein, means a physically discrete unit suitable for use as a single dose in the subject to be treated; each unit contains a predetermined amount of the active compound calculated to produce the desired therapeutic effect in association with the desired drug carrier. The specification of the unit dosage form in the disclosure is determined by the unique characteristics of the active compound and the specific therapeutic effect to be achieved, as well as the inherent limitations in the field of mixing such active compounds for the treatment of individuals. For example, a unit dose may be a certain amount of carrier genome, or a certain amount of carrier genome per milliliter, or a unit dose of a drug composition measured using the multiplicity of infection (MOI), which refers to the ratio or multiple of the vector or viral genome to the cell to which the nucleic acid can be delivered.

Pharmaceutical compositions may be included in containers, packages, or dispensers, such as injection syringes, along with the instructions for administration.

### Delivery of recombinant adeno-associated virus (rAAV)

The disclosure relates to a method for preventing or treating a disease or condition, wherein the method comprises the delivery to a subject of a therapeutically effective amount of the rAAV virus or pharmaceutical composition of the invention. The rAAV of the present invention may be delivered to the subject by any suitable method known in the art. For example, it is preferable to administer rAAV suspended in a physiologically compatible carrier (e.g., in a composition) to a subject, i.e. a host animal such as a human, mouse, rat, cat, dog, sheep, rabbit, horse, cow, goat, swine, cavy, hamster, chicken, turkey, or non-human primate (e.g. macaque). In some embodiments, the host animal does not include human. In some embodiments, the subject is human.

"Therapeutic effective dose" refers to the amount that is effective for the dose and time required to achieve the desired therapeutic effect. The therapeutic effective amount of rAAV virus or drug composition may vary based on factors such as the disease state, age, sex, and weight of the subject to be treated, and the ability of the rAAV virus or drug composition to trigger the desired response in the subject. The dosing regimen can be adjusted to provide an optimal therapeutic response. The therapeutic effective dose is also usually the amount in which any toxic or harmful effects of the rAAV virus or drug composition are outweighed by the beneficial effects of the treatment. "Preventive effective dose" refers to the amount that is effective in the dose and time required to achieve the desired preventive effect, such as preventing or suppressing various conditions. Prophylactic doses may be used in subjects before or in the early stages of the disease, and in some cases the prophylactic effective dose may be greater or less than the therapeutic effective dose. The dose is largely dependent on the condition and size of the subject being treated as well as the treatment formulation, frequency of treatment and route of administration. The regimen for ongoing treatment, including dose, formulation, and frequency, may be guided by initial response and clinical judgment.

In some embodiments, subject is given the rAAV virus or drug composition once a day, once a week, once a fortnight, once a month, once every two months, once every three months, once every six months, once a year or once every two years, once every five years, once a lifetime.

Example of dosing and delivery route comprises intravenous (I.V.), intra-articular, intra-peritoneal (I.P.), intra-arterial, intramuscular, parenteral, subcutaneous, intra-pleural, dermal, transdermal, parenteral, such as transmucusal, intra-cranial, intra-spinal, oral (digestive), mucosal, respiratory, intra-nasal, intubation, intra-pulmonary, intra-pulmonary infusion, buccal, sublingual, intravascular, intrathecal, intracavitary, iontherapy, intraocular, intraglandular, intraorgan, intrafallopian.

In some embodiments, rAAV delivery to mammalian subject may affect the mammalian eye by, for example, intraocular injection, subretinal injection, suprachoroidal injection (for example, suprachoroidal space injection) or topical administration (for example, eye drops), or by injection into the affected eye tissue to affect the eye of mammalian animals (such as intravitreal injection). "Eye tissue" refers to any tissue derived from or contained in the eye. Unrestricted examples of eye tissue include neurons, retina (e.g., photoreceptor cells), sclera, choroid, retina, vitreous body, macula, fovea centralis, optic disk, crystalline lens, pupil, iris, fluid, cornea, conjunctival ciliary body, and optic nerve. The retina is located at the back of the eye, which consists of photoreceptor cells. These photoreceptor cells (such as rods and cones) give vision by distinguishing colors and contrast in the visual field. In some embodiments, the rAAV or composition in the disclosure is administered by intraocular injection. In some embodiments, the rAAV or composition in the disclosure is administered by intravitreal injection. In some embodiments, the rAAV or composition in the disclosure is administered by subretinal injection. In some embodiments, the rAAV or composition in the disclosure is administered by suprachjoroidal (e.g., suprachoroidal space) injection.In some embodiments, the rAAV or composition in the disclosure is administered by intravenous injection.

In some embodiments, rAAV delivery to mammalian subjects may by, for example, intramuscular injection into the mammalian subjects.

In some embodiments, rAAV delivery to mammalian subjects may by, for example, intravenous injection into the mammalian subjects.

In some embodiments, rAAV delivery to mammalian subjects may by, for example, intra-articular injection into the mammalian subjects. "Intra-articular injection" is defined here as an injection or infusion into the joint. Intra-articular injections are commonly used to administer drugs to joints affected by inflammation.

The effect of administration of rAAV or pharmaceutical compositions of the disclosure may be preventing disease development, stopping disease development, reversing disease development, etc. Diseases or conditions are related to the target genes loaded in rAAV and the gene products expressed. For example, when the gene product is a VEGF antagonist, the prevented or treated disease or condition can be a VEGF-related disease. For example, "VEGF-related disease" refers to a group of diseases associated with abnormal VEGF activity/signaling. Many studies have shown that abnormal excess of VEGF can stimulate and induce pathological angiogenesis, causing angiogenesis related oculopathy. Nonrestrictive examples of angiogenesis related oculopathy include angiogenesis-dependent cancers, angiogenesis-related oculopathy, solid tumors (e.g., lung cancer, breast cancer, kidney cancer, liver cancer, pancreatic cancer, head and neck cancer, colon cancer, melanoma), blood-derived tumors (e.g., leukemia, metastatic tumors), benign tumors (e.g., hemangioma, acoustic neuroma, neurofibroma, trachitis and pyogenic granuloma), rheumatoid arthritis, psoriasis, red and swollen, Osier-Webber syndrome, myocardial angiogenesis, plaque, telangiectasia, hemophiliac joint tumor or angiofibroma.

In some embodiment, angiogenesis-related oculopathy includes, but are not limited to, diabetic retinopathy, retinopathy of prematurity, macular degeneration, corneal graft rejection, neovascular glaucoma and retrolental fibroplasia, epidemic keratoconjunctivitis, vitamin A deficiency, contact lens excessive wear, atopic keratitis, bacterial keratitis, ulcers, ulcers, bacterial keratitis, ulcers, primary keratosis, rheumatoid arthritis, systemic lupus, polyarteritis, trauma, Wegenus sarcoidosis, scleritis, Stephen Johnson's disease, Pemphigoid radial keratotomy, corneal graft rejection, sickle cell anemia, sarcoidosis, pseudoxanthoma elasticum, Pagetz's disease, venous occlusion, arterial occlusion, carotid artery obstructive disease, chronic uveitis/hyaluritis, mycobacterial infection, Lyme disease, systemic lupus erythematosus, retinopathy of prematurity, Eales disease, Behset's disease, retinitis or choroiditis infection, presumed histoplasmosis of the eye, Bests disease, myopia, optic pit, Stargardt's disease, planitis, chronic retinal deretination, hypervisemia syndrome, toxoplasmosis, trauma or post-laser complications.

In some embodiments, the oculopathy is selected from retinal neovascularization, choroidal neovascularization, iridal neovascularization, corneal neovascularization oculopathy, non-infectious uveitis, or glaucoma.

In some embodiments, the oculopathy were selected from age-related macular degeneration, macular oedema, diabetic macular oedema, macular oedema secondary to retinal vein occlusion, retinal vein occlusion, central retinal vein occlusion, branch retinal vein occlusion, macular oedema caused by branch retinal vein occlusion, diabetic retinal oedema, diabetic retinopathy, proliferative diabetic retinopathy, diabetic retinal ischemia, polypoidal choroidal vasculopathy, choroidal neovascularization secondary to degenerative myopia, or retinopathy of prematurity. Specific forms of macular degeneration may comprise acute macular degeneration, non-exudative age-related macular degeneration, and exudative age-related macular degeneration.

For example, when the gene product is a TNF- α antagonist, the disease or condition to be prevented or treated may be an arthritis disease or a related condition. Currently, it is estimated that there are more than 100 different forms of arthritis. In general, joint disorders are called arthropathy, and when inflammation of one or more joints is involved, this disorder is called arthritis. Herein, arthritis is construed as "joint pain" or "joint disease". In a preferred embodiment, arthritic disease is selected from adult onset Still disease, ankylosing spondylitis, arthritis, dorsalgia, Behget disease, blunt trauma, bursitis, calcium phosphate disease (CPPD), carpal tunnel syndrome, chondromalacia patella, chronic fatigue syndrome, complex local pain syndrome, cryopyrin-associated periodic syndrome (CAPS), degenerative disc disease, developmental dysplasia of hip, Eller-Danlos, familial Mediterranean fever, fibromyalgia, fifth disease, giant cell arteritis, gout, hemochromatosis, infectious arthritis, inflammatory arthritis, inflammatory bowel disease, joint replacement, juvenile arthritis, juvenile dermatomyositis (JD), juvenile idiopathic arthritis (JIA), juvenile rheumatoid arthritis, juvenile scleroderma, Kawasaki disease, lupus, lupus in children and adolescents, Lyme disease, mixed connective tissue disease, myositis (including polymyositis, dermatomyositis), osteoarthritis (OA), osteoporosis, pagets, gyratory rheumatism, patellofemoral pain syndrome, childhood rheumatic disease, childhood SLE, polymyalgia rheumatica, pseudogout, psoriatic arthritis, Rayno's phenomenon, reactive arthritis, reflex sympathetic dystrophy, Wright syndrome, rheumatic fever, rheumatism, rheumatoid arthritis, scleroderma, septic arthritis, xerosis, spinal stenosis, spondyloarthritis, Steele's disease, systemic juvenile idiopathic arthritis and systemic lupus erythematosus, systemic lupus erythematosus in children and adolescents, systemic sclerosis, temporal arteritis, tendonitis, vasculitis and Wegener granuloma.

In another preferred embodiment, arthritis diseases are selected from rheumatoid arthritis, juvenile rheumatoid arthritis, osteoarthritis, gout, pseudogout, spondylitis, Crohn's disease, plaque parapsoriasis, psoriatic arthritis, ankylosing spondylitis, septic arthritis, arthritis, juvenile idiopathic arthritis, blunt trauma, joint replacement or Still's disease.

In more preferred embodiments, the arthritic disease is a joint disorder involving inflammation of one or more joints. Preferably, the arthritis disease is selected from rheumatoid arthritis (RA), juvenile rheumatoid arthritis, osteoarthritis (OA), gout, pseudogout, spondyloarthritis (SpA), psoriatic arthritis, ankylosing spondylitis, septic arthritis, arthritis, juvenile idiopathic arthritis, and Still's disease.

The present invention is further described below in conjunction with examples, but these examples do not constitute any limitation of the present invention.

The experimental methods used in the following examples are conventional unless otherwise specified.

Materials, reagents, etc. used in the following examples may be obtained commercially unless otherwise specified.

### Examples 1 Construction of mutants

Mutant of AAV8 was generated by using site-directed mutagenesis and recombinant DNA techniques. The mutant comprised a polypeptide substitution of 5-14 amino acids located at sites Q588-A592 (QQNTA) of the parent AAV8, or further comprised a mutation corresponding to amino acids N262-N272 of parent AAV8 capsid protein, wherein the mutated amino acid sequence was SQSGASNDNH (SEQ ID NO: 10), or further comprised D80N, V125A, V125G mutations, as shown in Table 1. The schematic diagram of the exemplary No. 12 capsid mutant plasmid is shown in Figure 2.

**Table 1**

| **mutant** | **Q588-A592 amino acid substitution** |
|---|---|
| No. 12 | RGNRQ (SEQ ID NO: 1) |
| No. 121 | QQNTARGNRQ (SEQ ID NO: 2) |
| No. 122 | RGNRQAAQQNTA (SEQ ID NO: 3) |
| No. 123 | RGNRQQNTA (SEQ ID NO: 4) |
| No. 124 | RGNRQQQNTA (SEQ ID NO: 5) |
| No. 125 | SGNTQ (SEQ ID NO: 6) |
| No. 128 | RGNQQNTARQ (SEQ ID NO: 7) |
| No. 129 | RGNQQPRPTSRQ (SEQ ID NO: 8) |
| No.1213 | RGNRQAAQQPTPTS (SEQ ID NO: 9) |
| No.1214 | RGNRQQQPTPTS (SEQ ID NO: 19) |

**Table 2**

| **mutant** | **Q588-A592 amino acid substitution** | **D80 mutation** | **V125 mutation** |
|---|---|---|---|
| No.1281 | RGNQQNTARQ (SEQ ID NO: 7) | D80N | V125A |
| No. 1282 | RGNQQNTARQ (SEQ ID NO: 7) | D80Q | V125G |

**Table 3**

| **mutant** | **Q588-A592 amino acid substitution** | **N262 to N272 mutations** |
|---|---|---|
| No. 1312 | RGNRQ (SEQ ID NO: 1) | SQSGASNDNH (SEQ ID NO: 10) |

Another group of capsid protein mutants inserted a polypeptide sequence after amino acid Q589 of parent AAV8, as shown in Table 4.

**Table 4**

| **variant** | **Inserted polypeptide sequence after site of 589 amino acid** |
|---|---|
| No. 15 | RGDLTTPQQ (SEQ ID NO: 20) |
| No.151 | RGDLNTPQQ (SEQ ID NO: 21) |
| No. 152 | RGDVSSPQQ (SEQ ID NO: 22) |

The virus package of the AAV8 mutant was generated by co-transfection of three plasmids: the first plasmid (pAAV-CAG-EGFP) comprising expression cassette of target gene (GFP or luciferase) with side-joined ITR; the mutant plasmid (second plasmid) encoding Rep/Cap genes constructed in this example 1; and the third plasmid containing adenovirus auxiliary function genes. After transfection, the cells were collected, and lysed to release the virus, and the virus titer was determined by PCR, reserved for followed-up progress.

### Example 2 Transfection experiments by intravitreal injection in mice

### 2.1 EGFP mRNA expression level of mutant

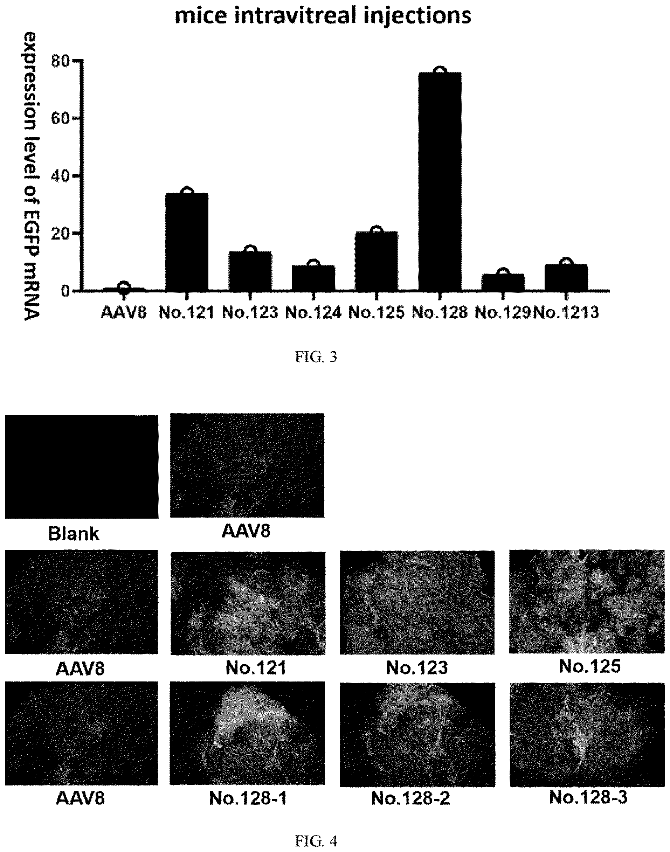

The purified AAV8 mutant viruses were administrated to mice via intravitreal injection at 7E+8vg/each eye. 21 days after intravitreal injection, mice eyeballs were extracted, and were ground using a freezing homogenizer. RNA was extracted using a total RNA extraction kit, and cDNA was synthesized using a reverse transcription kit. The specific EGFP primer probe set was determined by qPCR (wherein, the sequence of upstream primer EGFP-F was: 5'-CACATGAAGCAGCACGACTT-3' (SEQ ID NO: 26); the sequence of downstream primer EGFP-R was: 5'-TCGTCCTTGAAGAAGATGGT-3' (SEQ ID NO: 27); the probe was: 5'-AGTCCGCCATGCCCGAAGGCT-3'-TAMRA, and the sequence contained in the probe was shown in SEQ ID NO: 28). The normalized EGFP mRNA level of AAV8 variants were calculated, that is, the effect of different AAV8 variants on vitreous transfection was compared, and the results were shown in Figure 3.

### 2.2 Retinal patching

The purified AAV8 mutant viruses were administrated to mice via intravitreal injection at 7E+8vg/ each eye. 21 days after intravitreal injection, mice eyeballs were extracted, fixed overnight with 4% paraformaldehyde, and mice retinas were separated under a standing microscope by surgical microscope snips, the mice retinas were patched with slices, and EGFP fluorescence signals were captured by confocal shooting, the results were shown in Figure 4.

### Example 3 Transfection experiments by intravitreal injection in rats

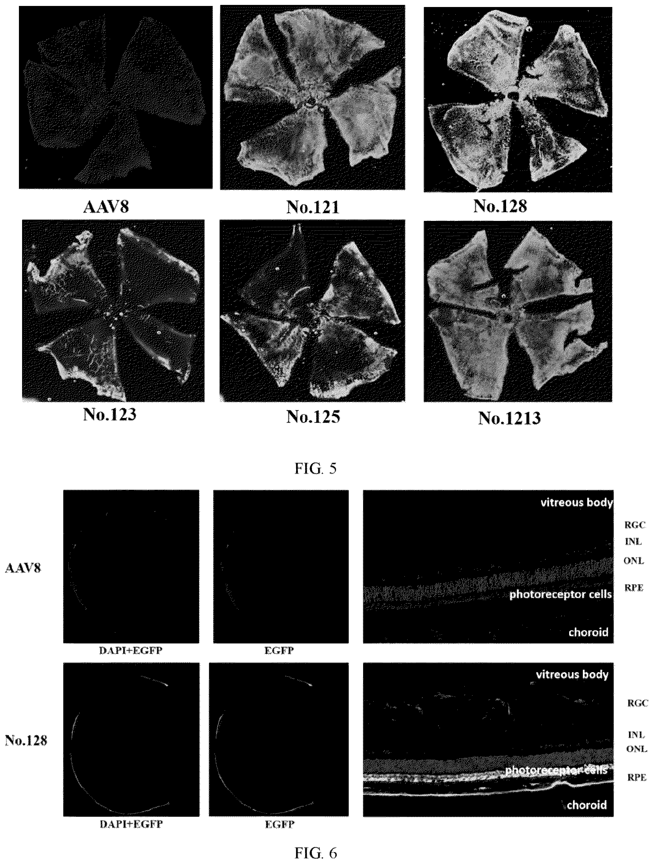

The purified AAV8 mutant viruses were administrated to rats via intravitreal injection at 1.92E +9vg/ each eye; 21 days after intravitreal injection, the eyeballs of rats were extracted; the eyeballs of rats were fixed with 4% paraformaldehyde overnight; the retina of the rats was separated by surgical microscope scissors under the upright microscope; the retinal of the rats were patched and the EGFP fluorescence signal were captured by the fluorescence scanning microscope, and the results were shown in Figure 5.

### Example 4 Transfection experiments by suprachorioidal space injection in rabbits

The purified AAV8 mutant viruses were administrated to New Zealand rabbits viah suprachorioidal space injection at 5E+10vg/ each eye; the eyeballs of New Zealand rabbits were extracted 14 days after suprachorioidal space injection; New Zealand rabbit eyeballs were fixed with 4% paraformaldehyde overnight; the fixed eyeballs were treated with dehydration and tissue embedding; freezing section was performed, followed by nuclear staining (DAPI); confocal microscopy was used for full image scanning to capture EGFP fluorescence signal and DAPI signal, and the results were shown in Figure 6 (in Figure 5, EGFP: self-fluorescence, DAPI: nucleus, RGC: ganglion cells, INL: retinal inner layer; ONL: outer nuclear layer of retina; RPE: retinal epithelial cells), according to the fluorescence figure, the transduction efficiency of wild type on photoreceptors and RPE layer was not high, while that of mutants on photoreceptor and RPE layer was greatly improved.

### Example 5 ARPE19 cell infection experiment

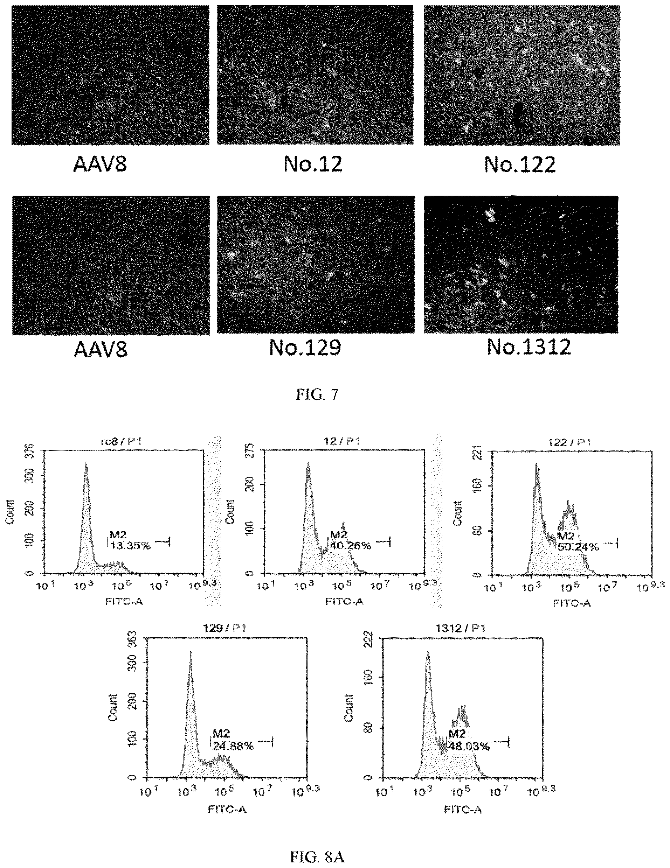

ARPE19 cells were planted in 12-well plates and cultured until the cell fusion degree was 70-80% (generally 1.0E+5 cells); virus supernatant (4.5E+9 Vg) was added in the desired ratio and the amount of virus supernatant + total medium was made being 1000 µL; after 16 h of infection, the medium was replaced every day and the culture was continued until 72 h; the fluorescence signal of EGFP was captured by confocal shooting, and the expression of fluorescent protein was observed. The 48-hour infection of ARPE19 cells was shown in Figure 7.

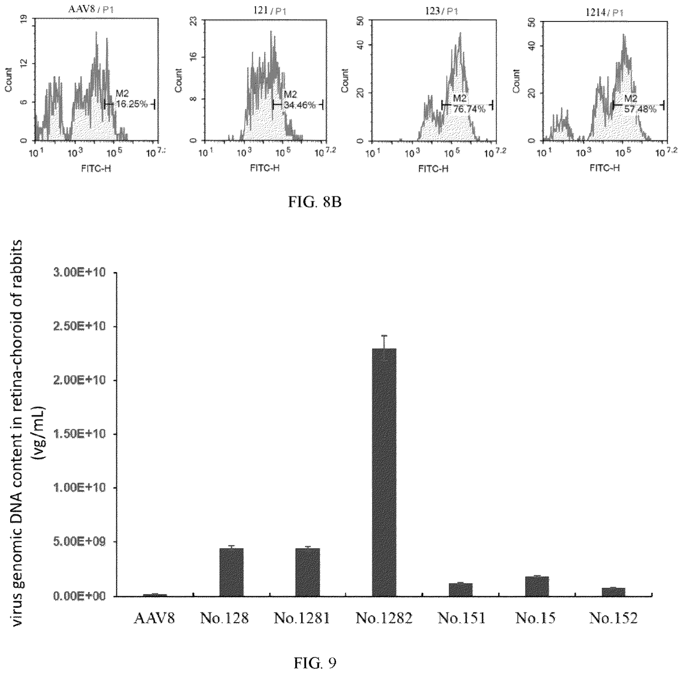

Meanwhile, number of EGFP positive cells was counted by flow cytometry. Cells were cultured in a 6-well plate cell dish with 2E+5 cells, 1E+10 vg packed mutant virus was added and cultured at 37°C for 48 h; the cells were digested with trypsin, and were collected and re-suspended with 300 µL sterile PBS; FITC (488nm) channel was used for measurement by Roche flow cytometry, in which the total number of measured cells was set to 100,000 and the number of EGFP positive cells was counted. The results were shown in Figure 8A and 8B.

### Example 6 Transfection experiment by suprachorioidal space injection in rabbits

The different mutant viruses AAV-mCherry (mCherry is a protein isolated from corals and used here as a red fluorescent dye tracer) were prepared by the tri-plasmid co-transfection method as described in Example 1, and the genomic titer of each virus was measured by ddPCR; the genome titer of each mutantt virus was diluted to 1.0E+12 vg/mL by buffer solution, and suprachorioidal injection was perfomed 4 mm above the temporal of rabbit eyes with 100 µL in each eye, 2 rabbits and 4 eyes were injected with each virus; 5 days later, New Zealand rabbits were executed, eyeball was extracted, after retinal/choroid tissue was separated, tissue homogenization was carried out at low temperature, supernate was taken, viral genomic DNA was extracted by viral genome DNA extraction kit, and the relative content of retinal/choroid virus genomic DNA was determined by qPCR for each virus, the results were shown in Table 5 and Figure 9.

**Table 5**

| | AAV8 | No. 128 | No.1281 | No. 1282 | No.151 | No. 15 | No.152 |
|---|---|---|---|---|---|---|---|
| Viral genomic DNA relative content (vg/mL) | 1.88 E8 | 4.45 E9 | 4.41 E9 | 2.30 E10 | 1.19 E9 | 1.81 E9 | 7.71 E8 |

### Example 7 Protein expression experiment in APRE infected cells

AAV8-Aflibercept virus and AAV No. 12-Aflibercept mutant virus were prepared by the tri-plasmids co-transfection method as described in Example 1, and the genomic titers of each virus was measured by ddPCR; the virus genomic titer of each mutant was diluted to 3.0E12 vg/mL with 3.0E5 cell count by buffer solution, 10 µL virus was added to each well, the medium was changed after 16h culture, and cells and cell supernatant were collected after 72 h; after freezing with liquid nitrogen, repeated freezing and thawing for 3 times, the cells were lysed, and the expression level of Trap protein was determined by ELISA. The results showed that the protein expression of AAV8- Aflibercept virus was 97 ng/ml, and that of AAV No. 12-Aflibercept virus was 1662 ng/ml.

### Example 8 Protein expression experiment in Rhesus after suprachorioidal injection

AAV No. 128-Aflibercept virus was prepared by the tri-plasmids co-transfection method as described in Example 1, and the genomic titer of each virus was measured by ddPCR; the genomic titer of the virus was diluted to 1.0E13 vg/mL by buffer solution; the virus was injected into the suprachoroidal space 4 mm above the temporal part of the rhesus's eye with 100 µL in each eye. One monkey and two eyes were injected; 56 days later, the rhesus were executed, the eyeball was extracted, and various tissues of the monkey's eye (including aqueous humor, choroid, conjunctiva, iridociliary, retina, sclera, vitreous body) were separated, after adding protease inhibitors, the tissue homogenization was carried out at low temperature, the supernate was taken, and the expression level of Aflibercept protein (ng/ml) was determined by ELISA, the results were shown in Table 6.

**Table 6**

| No. | Aqueous humor | choroid | conjunctiva | iridociliary | retina | sclera | Vitreous body |
|---|---|---|---|---|---|---|---|
| Left eye | 12.1 | 1638 | 48.48 | 134.4 | 4056 | 48.42 | 44.9 |
| Right eye | 9.95 | 1032 | BLQ* | 502.2 | 2112 | 84 | 36 |

| | | | | | | | |
|---|---|---|---|---|---|---|---|
| BLQ* : Not detected. | | | | | | | |

### Example 9 Transfection experiment in mice by intramuscular injection

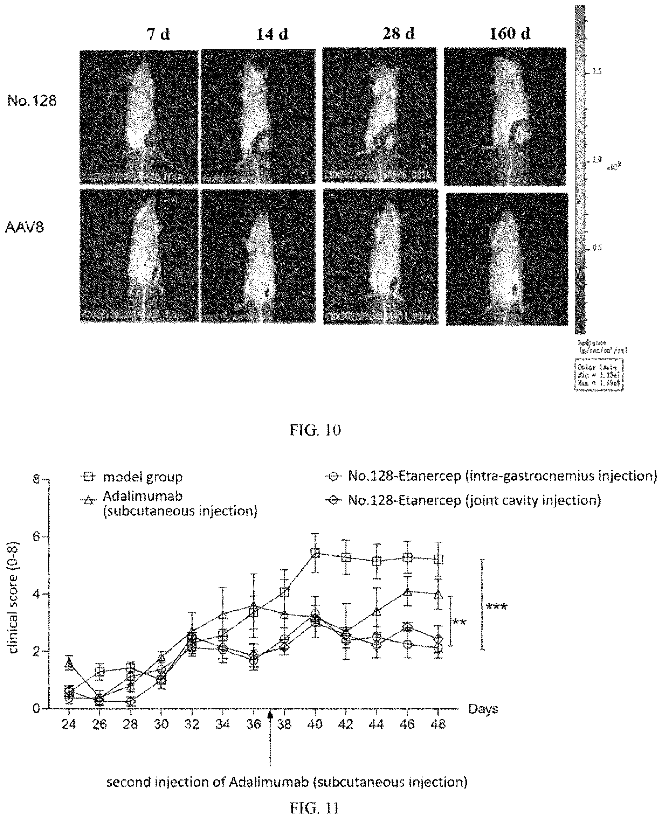

AAV8-luciferase and AAV No. 128-luciferase viruses were prepared by the tri-plasmids co-transfection method as described in Example 1, and the genomic titers of the viruses were measured by ddPCR; the virus genomic titer was diluted to 1.62E11 vg/mL by buffer solution and injected into the gastrocnemius of the right leg of mice with a volume of 50 µL, and 5 mice (Balb/c) were injected with each virus. On days 7, 14, 28 and 160 after virus injection, a certain amount of sodium fluorescein substrate was injected into the abdominal cavity of mice respectively, and mice in vivo imaging were performed, fluorescence intensity was analyzed by in vivo imager, the results were shown in Table 7 and Figure 10.

**Table 7**

| **No.** | **7d** | **14 d** | **28 d** | **160 d** |
|---|---|---|---|---|
| AAV8-luciferase | 6.69 E+06 | 6.30 E+06 | 5.93 E+06 | 4.76 E+06 |
| AAV No.128-luciferase | 1.49 E+08 | 3.18E+08 | 3.72 E+08 | 1.74 E+08 |

### Example 10 Protein expression experiment in transfected 293F cells

The plasmids of AAV-Etanercept and AAV-Adalimumab were constructed respectively; 2µg plasmid was selected and single-plasmid transfection was performed with 293F cells, 72 h later, the protein expression level of the plasmid was detected by ELISA. The results showed that the expression level of AAV-Adalimumab plasmid was 1010 ng/mL, while the expression level of AAV -Etanercep plasmid was 2020 ng/mL.

### Example 11 Effectiveness experiment of joint cavity/intramuscular injection in mice

### 1) Sample solution preparation

Test sample preparation: AAV No. 128- Etanercep virus was prepared by the method described in Example 1 and the viral genomic titer was determined, then diluted to 9.98E12 vg/mL, and set aside.

Positive control: Adalimumab original solution was extracted 40 mg/400 µl and diluted to 1.6 µg/µl.

### 2) Modeling

First modeling on day 0:6-week-old DBA/1 male mice were selected, firstly 0.6ml bovine type II collagen (CII) solution (2 mg/ml) and 0.6ml (4 mg/ml) of complete Froman's adjuvant were loaded into the film extruder to prepare about 1 ml of milky emulsion. 100 µl emulsion was injected subcutaneously into each of mice by tail root. Swelling of the tail root was observed, and the basic success of administration was indicated by the observation of the emulsion did not leak out, and the mice did not die.

Second modeling on the 21st day: 0.6ml bovine type II collagen (CII) (2mg/ml) and 0.6ml (4 mg/ml) of incomplete Froman's adjuvant were put into the film extruder to prepare about 1 ml of milky emulsion, and 100 µl of emulsion was injected subcutaneously into each of mouse by tail root.

### 3) Administration

On day 22, AAV No. 128-Etanercept virus sample solution 10 µl/ankle was administrated via joint cavity injection (injected into both legs).

On day 22, AAV No. 128-Etanercept virus sample solution 100 µl/ankle was injected into the muscle near the joint cavity (injected into both legs).

On day 22, the adalimumab group was administrated 160 µg adalimumab subcutaneously on the back (the 40mg/400 µl original solution was diluted to 1.6 µg/µl, and 100 µl was injected subcutaneously on the back with an insulin needle), once every two weeks.

### 4) Result observation

From day 23, the second day of administration, the thickness of the sole of the foot was monitored and measured by using an electronic vernier caliper and a clinical score of joint inflammation was performed every two days. The scoring criteria are shown in Table 8:

**Table 8**

| **Severity score** | **Degree of inflammation** |
|---|---|
| 0 | No signs of erythema or swelling |
| 1 | Erythema and mild swelling limited to the tarsal or ankle joints |
| 2 | Erythema and mild swelling from ankle to tarsal bone |
| 3 | Erythema and moderate swelling of the joints from ankle to metatarsal |
| 4 | Erythema and severe swelling around the ankles, feet and fingers, or stiffness of the limbs |

### 5) Results

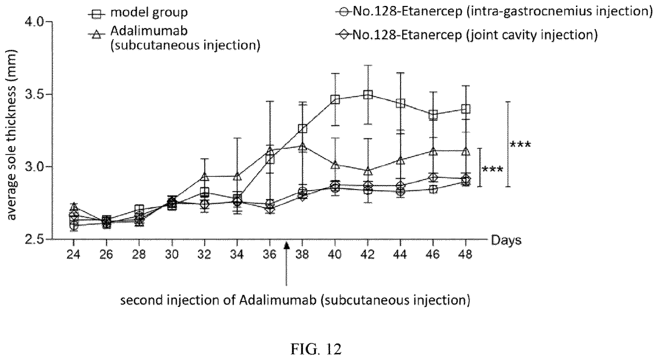

The results of sole thickness measurement and the clinical score of the inflammation of the joint are shown in Figure 11 and Figure 12.

The above embodiments express only several embodiments of the invention, in which the description is more specified and detailed, but it cannot be understood as a limitation of the patent scope of the invention. It should be noted that for ordinary skilled person in the field, a number of deformation and improvement can be made without deviating from the concept of the invention, which are within the scope of protection of the invention. Therefore, the scope of protection of the invention patent shall be subject to the attached claims.

## Claims

1. A modified adeno-associated virus (AAV) capsid protein **characterized in that** the capsid protein comprises a polypeptide substitution of approximately 5-14 amino acids relative to the parent AAV capsid protein and wherein an AAV comprising the modified capsid protein has enhanced retinal cell infectivity when compared to the AAV comprising the corresponding parent AAV capsid protein.

2. The capsid protein according to claim 1, **characterized in that** the polypeptide comprises amino acid sequence selected from RGNRQ (SEQ ID NO: 1), QQNTARGNRQ (SEQ ID NO: 2), RGNRQAAQQNTA (SEQ ID NO:3), RGNRQQNTA (SEQ ID NO: 4), RGNRQQQNTA (SEQ ID NO: 5), SGNTQ (SEQ ID NO: 6), RGNQQNTARQ (SEQ ID NO:7), RGNQQPRPTSRQ (SEQ ID NO: 8), RGNRQAAQQPTPTS (SEQ ID NO: 9) or RGNRQQQPTPTS (SEQ ID NO: 19); and the substitution is located at amino acid sites 588-592 of parent AAV8 or at the corresponding position of another serotype capsid protein.

3. The capsid protein according to claim 2, **characterized in that** the polypeptide comprises amino acid sequence selected from RGNRQ (SEQ ID NO: 1), QQNTARGNRQ (SEQ ID NO: 2), RGNRQAAQQNTA (SEQ ID NO:3), RGNRQQNTA (SEQ ID NO: 4), SGNTQ (SEQ ID NO: 6), RGNQQNTARQ (SEQ ID NO: 7) or RGNRQQQPTPTS (SEQ ID NO: 19).

4. The capsid protein according to claim 2 or 3, **characterized in that** the capsid protein further comprises a mutation at amino acid sites 262-272 of the parent AAV8 capsid protein or at the corresponding position of another serotype capsid protein, resulting in an amino acid sequence of SQSGASNDNH (SEQ ID NO: 10).

5. The capsid protein according to any one of claims 1-4, **characterized in that** the capsid protein comprises a polypeptide substitution at amino acid sites 588-592 of parent AAV8 or at the corresponding position of another serotype capsid protein, in which the polypeptide is RGNRQ (SEQ ID NO: 1); and a mutation at amino acid sites 262-272 of the parent AAV8 capsid protein or at the corresponding position of another serotype capsid protein, resulting in an amino acid sequence of SQSGASNDNH (SEQ ID NO: 10).

6. The capsid protein according to any one of claims 1-5, **characterized in that** the AAV serotype is selected from AAV1, AAV2, AAV3, AAV4, AAV5, AAV6, AAV7, AAV8, AAV9, AAV10, AAV11, AAV12, AAV-DJ, AAV-DJ8, AAV-DJ9, AAVrh8, AAVrh8R, and AAVrh10.

7. The capsid protein according to claim 6, **characterized in that** the AAV serotype is AAV8.

8. The capsid protein according to any one of claims 1-7, **characterized in that** the capsid protein further comprises an amino acid mutation at the site of D80 and/or V125 relative to the parent AAV8 capsid protein.

9. The capsid protein according to claim 8, **characterized in that** the mutation is D80N and/or V125A.

10. The capsid protein according to claim 8, **characterized in that** the mutation is D80Q and/or V125G.

11. The capsid protein according to claim 8 or 9, **characterized in that** the capsid protein comprises a polypeptide substitution at amino acid sites 588-592 of the parent AAV8, in which the polypeptide is RGNQQNTARQ (SEQ ID NO: 7); and amino acid mutations at sites of D80 and V125 relative to parent AAV8 capsid proteins, in which the mutations are D80N and V125A.

12. The capsid protein according to claim 8 or 10, **characterized in that** the capsid protein comprises a polypeptide substitution at amino acid sites 588-592 of the parent AAV8, in which the polypeptide is RGNQQNTARQ (SEQ ID NO: 7); and amino acid mutations at sites of D80 and V125 relative to parent AAV8 capsid proteins, in which the mutations are D80Q and V125G.

13. A modified adeno-associated virus (AAV) capsid protein **characterized in that** the capsid protein comprises a polypeptide insertion after amino acid 589 of parent AAV8 or at the corresponding position of another serotype capsid protein, and wherein the AAV virus comprising the modified capsid protein has enhanced retinal cell infectivity, when compared to an AAV virus comprising the corresponding parent AAV capsid protein.

14. The capsid protein according to claim 13, **characterized in that** the polypeptide comprises amino acid sequence selected from RGDLTTPQQ (SEQ ID NO: 20), RGDLNTPQQ (SEQ ID NO: 21) and RGDVSSPQQ (SEQ ID NO: 22).

15. The capsid protein according to claim 13 or 14, **characterized in that** the AAV serotype is selected from AAV1, AAV2, AAV3, AAV4, AAV5, AAV6, AAV7, AAV8, AAV9, AAV10, AAV11, AAV12, AAV-DJ, AAV-DJ8, AAV-DJ9, AAVrh8, AAVrh8R, and AAVrh10.

16. The capsid protein according to claim 15, **characterized in that** the AAV serotype is AAV8.

17. A recombinant adeno-associated virus (rAAV), comprising:
i. modified capsid proteins according to any one of the preceding claims 1-16;
ii. heterologous nucleic acids comprising encoded gene products.

18. The recombinant adeno-associated virus according to claim 17, **characterized in that** the gene product is a VEGF antagonist or a TNF-α antagonist.

19. The recombinant adeno-associated virus according to claim 18, **characterized in that** the VEGF antagonist is selected from Aflibercept, Combercept, Ranibizumab, and Brolucizumab.

20. The recombinant adeno-associated virus according to claim 19, **characterized in that** the VEGF antagonist is selected from Aflibercept.

21. The recombinant adeno-associated virus according to claim 18, **characterized in that** the TNF- aantagonist is selected from: Etanercept, Infliximab, Adalimumab, Pecelizumab, Golimumab.

22. The recombinant adeno-associated virus according to claim 21, **characterized in that** the TNF-α antagonist is Etanercept.

23. A pharmaceutical composition, comprising:
a) recombinant adeno-associated virus according to any one of claims 17-22;
b) pharmaceutically acceptable excipients.

24. Use of the recombinant adeno-associated virus according to any one of claims 17-22 in the preparation of drugs for prevention or treatment of oculopathy.

25. A method for prevention or treatment of oculopathy, comprising administration of an effective amount of the recombinant adeno-associated virus according to any one of claims 17-22 or the pharmaceutical composition according to claims 23 to an individual in need.

26. The method according to claim 25, **characterized in that** the drug is administrated via intravitreal, subretinal, or suprachoroidal injection.

27. The method according to claim 26, **characterized in that** the drug is administrated via suprachoroidal space injection.

28. The use according to claim 24 or the method according to any one of claims 25-27, **characterized in that** the oculopathy is selected from retinal neovascularization, choroidal neovascularization, iridal neovascularization, corneal neovascularization oculopathy, non-infectious uveitis or glaucoma.

29. The use according to claim 24 or the method according to any one of claims 25-27, **characterized in that** the oculopathy is selected from age-related macular degeneration, macular oedema, diabetic macular oedema, macular oedema secondary to retinal vein occlusion, retinal vein occlusion, central retinal vein occlusion, branch retinal vein occlusion, macular oedema caused by branch retinal vein occlusion, diabetic retinal oedema, diabetic retinopathy, proliferative diabetic retinopathy, diabetic retinal ischemia, polypoidal choroidal vasculopathy, choroidal neovascularization secondary to degenerative myopia, or retinopathy of prematurity.

30. Use of the recombinant adeno-associated virus according to any one of the claims 17-22 in the preparation of drugs for prevention or treatment of arthritic diseases or related conditions.

31. A method for treatment of arthritic disease or related condition, comprising administration of an effective amount of a recombinant adeno-associated virus according to any one of claims 17-22 or a pharmaceutical composition according to claims 23 to an individual in need.

32. The method according to claim 31, **characterized in that** the drug is administrated via intravenous, subcutaneous, muscular or joint injection.

33. The method according to claim 32, **characterized in that** the drug is administrated via muscular or intra-joint injection.

34. The use according to claim 30 or the method according to any one of claims 31-33, **characterized in that** the arthritis disease or related condition is selected from rheumatoid arthritis, juvenile rheumatoid arthritis, osteoarthritis, gout, pseudogout, spondylitis, Crohn's disease, plaque parapsoriasis, psoriatic arthritis, ankylosing spondylitis, septic arthritis, arthritis, juvenile idiopathic arthritis, blunt trauma, joint replacement or Still's disease.
